# EUROPÄISCHE PATENTSCHRIFT

(11) **EP 2 203 210 B1**
(45) Veröffentlichungstag und Bekanntmachung des Hinweises auf die Patenterteilung: **12.06.2019**
(21) Anmeldenummer: 08834535.0
(22) Anmeldetag: 25.09.2008
(51) Int. Cl.: B05B 11/00, A61M 35/00, B05C 17/00, A61K 9/00, A61K 9/70

(54) **VERFAHREN ZUM APPLIZIEREN EINES FLÜSSIGEN, MEDIZINISCHEN BEHANDLUNGSMEDIUMS AUF EINEN ZU BEHANDELNDEN BEREICH SOWIE MEDIZINPRODUKT**
METHOD FOR APPLYING A LIQUID MEDICINAL TREATMENT MEDIUM TO AN AREA TO BE TREATED AND MEDICINAL PRODUCT
PROCÉDÉ D'APPLICATION D'UN MILIEU DE TRAITEMENT MÉDICAL LIQUIDE SUR UNE ZONE À TRAITER, ET PRODUIT MÉDICAL CORRESPONDANT

(30) Priorität: 27.09.2007 DE 102007046600
(43) Veröffentlichungstag der Anmeldung: 07.07.2010
(60) Teilanmeldung: 11005433.5
(73) Patentinhaber: Linhardt GmbH & Co. KG, 94234 Viechtach (DE); Temmler Pharma GmbH & Co. KG, 35039 Marburg (DE)
(72) Erfinder: SOLLINGER, Horst, 83620 Feldkirchen (DE); BEIL, Johann, 94234 Viechtach (DE)
(74) Vertreter: Glück Kritzenberger Patentanwälte PartGmbB
(86) Internationale Anmeldenummer: PCT/DE2008/001576
(87) Internationale Veröffentlichungsnummer: WO 2009/039838

(56) Entgegenhaltungen:
- WO-A-01/32319
- WO-A-97/29347
- US-A1- 2002 192 011
- US-A1- 2003 032 980
- US-A1- 2004 081 681
- US-A1- 2005 261 639
- US-B1- 6 283 933

## Beschreibung

Die Erfindung bezieht sich auf ein Medizinprodukt gemäß Oberbegriff des unabhängigen Patentanspruchs 1. Ein medizinisches Produkt dieser Art mit einem stiftförmigen Applikator (US 6 283 933 B1), der u.a. ein Applikatorgehäuse oder einen Behälter sowie eine Applikatorspitze an einem Ende des Behälters aufweist, ist bekannt. Für das Ausbringen eines antiviralen Wirkstoffes ist im Behälter eine diesen Wirkstoff aufnehmende Ampulle untergebracht. Der Wirkstoff wird aus der Ampulle mit der Applikatorspitze ausgebracht.

Aufgabe der Erfindung ist es, ein Medizinprodukt zu schaffen, mit dem ein besonders einfaches, gut dosiertes und vor allem auch genau lokalisiertes Aufbringen eines flüssigen medizinischen Behandlungsmediums auf einen zu behandelnden Bereich (Haut- oder Gewebebereich) eines menschlichen und/oder tierischen Körpers möglich ist. Zur Lösung dieser Aufgabe ist ein Medizinprodukt entsprechend dem Patentanspruch 1 ausgebildet.

Unter "medizinisches Behandlungsmedium" ist im Sinne der Erfindung ein Medium zu verstehen, welches in Lösung wenigstens einen medizinischen Wirkstoff enthält, wobei der wenigstens eine medizinische Wirkstoff ein Wirkstoff zur Behandlung von Warzen ist, und zwar durch äußere Anwendung.

Die den wenigstens einen Wirkstoff enthaltende Lösung ist beispielsweise eine ölige Lösung oder eine wässrige, alkoholische Lösung und dabei so ausgewählt, dass der erforderliche Fluss des Behandlungsmediums aus dem röhrchen- oder hülsenförmigen Behälter durch die Applikationsspitze an deren zum Applizieren dienendes Ende (Applikationsende) insbesondere auch ohne ein Zusetzen der Poren und/oder Kanäle innerhalb der Applikationsspitze gewährleistet ist, die Lösung aber auch die erforderliche Stabilität des Behandlungsmediums sicherstellt, insbesondere auch in physikalischer und/oder chemischer Hinsicht. Der verwendete Applikator weist beispielsweise die Größe eines üblichen Filzstiftes oder Markers auf. Es ist aber auch möglich, die Größe des Applikators und/oder das Volumen des zur Aufnahme des Behandlungsmediums dienenden Behälterinnenraumes oder Reservoirs dieses Applikators so klein zu wählen, dass das Behandlungsmedium lediglich für eine einmalige Behandlung oder aber nur für eine sehr begrenzte Anzahl von Behandlungen ausreichend ist.

Grundsätzlich erfolgt das Applizieren des Behandlungsmediums durch Bestreichen des zu behandelnden Bereichs mit der Applikationsspitze bzw. mit deren Applikationsende, wobei die Applikationsspitze bevorzugt aus einem für einen gesteuerten und dosierten Durchtritt des Behandlungsmediums geeigneten Material besteht, nämlich aus einem porösen Material, auch gesinterten Material, z.B. aus einem gesinterten Kunststoff.

In Weiterbildung der Erfindung ist das erfindungsgemäße Medizinprodukt beispielsweise so ausgebildet, dass das Behandlungsmedium wenigstens einen Wirkstoff in einer öligen Lösung enthält, wobei die ölige Lösung des Behandlungsmediums mindestens ein mineralisches Öl und/oder wenigstens ein Derivat eines mineralischen Öls enthält, z.B. Paraffinöl und/oder Silikonöl, und/oder das Behandlungsmedium oder dessen Lösung wenigstens ein pflanzliches Öl und/oder Ölprodukt enthält, welches aus wenigstens einem pflanzlichen Öl durch Umestern, Teilreduktion, Verethern und/oder Teilsynthese gewonnen ist, und/oder dass als pflanzliches Öl Sonnenblumenöl und/oder andere in den Arzneibüchern monographierte, pflanzliche Öle verwendet werden.

Gemäß einer bevorzugten Ausführungsform werden als Ölprodukt Glycerolester oder andere Ester von Festsäuren mit Fettalkoholen, Fettalkohole und/oder deren Ether und Ester verwendet, auch in beliebiger Mischung und in beliebigen Anteilen.

Gemäß einer bevorzugten Ausführungsform enthält die ölige Lösung zumindest einen Zusatz in Form von Tensiden, vorzugsweise mit einem Anteil von etwa 0,1 - 4,0 Gewichts-%, bezogen auf die Gesamtmasse der Lösung, und/oder in Form von Wachsen, vorzugsweise mit einem Anteil von etwa 0,1 - 1,0 Gewichts-% bezogen auf die Gesamtmasse der Lösung, und/oder Parfümöle und/oder Farbstoffe.

Gemäß einer bevorzugten Ausführungsform enthält das Behandlungsmedium den wenigstens einen medizinischen Wirkstoff in einer wässrigen, alkoholischen Lösung, wobei die wässrige alkoholische Lösung ein- oder mehrwertige Alkohole, wie z.B. Ethanol, Glycerol und/oder polymere Alkohole und/oder Isopropanol und/oder Propylenglykol und/oder PEG und/oder deren Ester und/oder Ether und/oder Ethanol und/oder Isopropanol in einer Konzentration von etwa 5,0 - 70 Gewichts-%, bezogen auf die Gesamtmasse der Lösung enthält.

Gemäß einer bevorzugten Ausführungsform enthält die wässrige alkoholische Lösung die mehrwertigen, gesättigten und/oder ungesättigten Alkohole und polymeren Alkohole, wie z.B. Propylenglykol und/oder deren Ester und/oder Ether in einer Konzentration von etwa 2,0 - 40 Gewichts-%, bezogen auf die Gesamtmasse der Lösung, und/oder die wässrige, alkoholische Lösung enthält den wenigstens einen medizinischen Wirkstoff in liposomaler und/oder nanosomaler Verkapselung, und/oder die wässrige alkoholische Lösung enthält einen Film bildenden Bestendteil, vorzugsweise einen Film bildenden polymeren Bestandteil, wobei als Film bildender Bestandteil beispielsweise Ethylacetat, vorzugsweise in einer Konzentration von etwa 5,0 - 40 Gewichts-%, und/oder beispielsweise Aceton, vorzugsweise in einer Konzentration von etwa 5,0 - 40 Gewichts%, jeweils bezogen auf die Gesamtmasse der Lösung, und/oder Polymere und/oder Co- und Crosspolymere, wie z.B. Polymethacrylate, vorzugsweise auch chemisch modifiziert verwendet werden.

Gemäß einer bevorzugten Ausführungsform enthält die wässrige alkoholische Lösung als Zusatz Tenside, vorzugsweise in einer Konzentration von etwa 0,5 - 5 Gewichts-%, bezogen auf die Gesamtmasse der Lösung, und/oder Fettalkohole und/oder deren Ester und Ether, vorzugsweise in einer Konzentration von etwa 0,5 - 4,0 Gewichts%, bezogen auf die Gesamtmasse der Lösung.

Gemäß einer bevorzugten Ausführungsform wird Polysobat 80 und/oder Octyldodecanol als Zusatz verwendet.

Gemäß einer bevorzugten Ausführungsform enthält die wässrige alkoholische Lösung als Zusatz Verdicker auf Basis von Cellulose und/oder Stärke, beispielsweise Oligosaccharide und/oder Polyacrylate, vorzugsweise in einer Konzentration von etwa 0,1 - 0,4 Gewichts-%, bezogen auf die Gesamtmasse der Lösung.

Gemäß einer bevorzugten Ausführungsform enthält die Lösung Parfümöle und/oder Farbstoffe.

Gemäß einer bevorzugten Ausführungsform besteht die Applikationsspitze aus einem porösen gesinterten Material, z.B. aus einem gesinterten polymeren Material.

Gemäß einer bevorzugten Ausführungsform ist die Applikationsspitze an ihrem zum Aufbringen des Behandlungsmediums auf die Haut dienenden Ende mit reduzierter Härte ausgebildet.

Gemäß einer bevorzugten Ausführungsform besteht die Applikationsspitze aus PE, PP, PVDF, PTFE und/oder EVA besteht, wobei die vorgenannten Merkmale jeweils einzeln oder in beliebiger Kombination verwendet sein können.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:
- Fig. 1: in einer Explosionsdarstellung die Elemente einer Vorrichtung zum Applizieren eines flüssigen medizinisch wirksamen Behandlungsmediums gemäß der Erfindung;
- Fig. 2: die Vorrichtung der Figur 1 im zusammengebauten Zustand;
- Fig. 3 u. 4: Darstellungen wie Figuren 1 und 2 bei einer weiteren Ausführungsform.

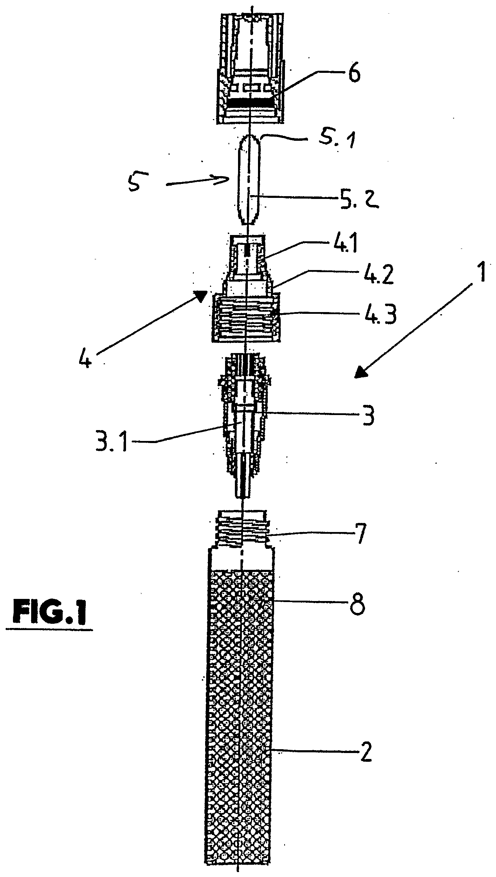

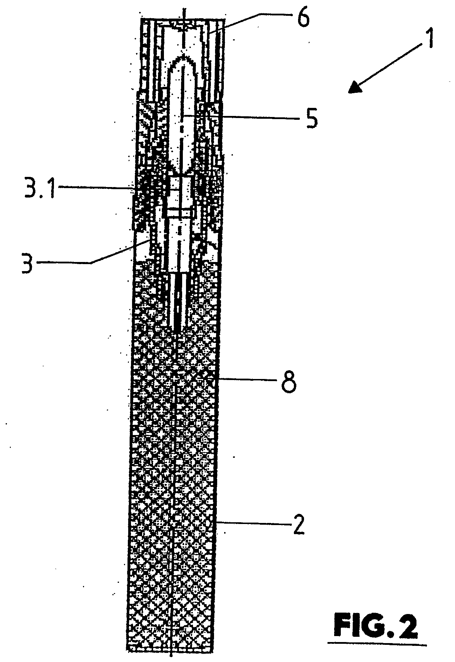

Die in den Figuren 1 und 2 allgemein mit 1 bezeichnete Vorrichtung (Applikator) besteht u.a. aus einem aus einem geeigneten Material, beispielsweise aus Kunststoff, Glas und/oder Metall hergestellten hülsen- oder röhrchenartigen, nur an einem Ende, d.h. bei der Darstellung der Figur 1 an seinem oberen Ende offenen Behälter 2 zur Aufnahme des Behandlungsmediums, aus einem eine Ventil- oder Dosierpumpe 3.1 enthaltenden Einsatz 3, aus einem hülsenartigen Mundstück 4 mit Abschnitten 4.1, 4.2 und 4.3 mit unterschiedlichem Innen- und Außendurchmesser aus einer Applikationsspitze 5 sowie aus einer Verschlusskappe 6, die ebenso wie zumindest Teile des Einsatzes 3 und des Mundstücks 4 als Formteil (Spritzgießteil) aus Kunststoff hergestellt ist.

Die Applikationsspitze 5 besteht aus einem porösen, Flüssigkeit leitenden Material und ist z.B. so ausgebildet, dass sie an ihrem im zusammengebauten Zustand des Applikators 1 aus dem Mundstück 4 vorstehenden Ende, d.h. an dem dortigen zum Applizieren des Behandlungsmediums dienenden Ende 5.1 (Applikationsende) weicher ausgeführt ist, als an ihrem übrigen Bereich bzw. Schaft 5.2. Weiterhin sitzt die Applikationsspitze 5 im Bereich ihres Endes 5.1 eine Porosität mit einer gegenüber dem Schaft 5.2 reduzierten Porengröße, d.h. die Porengröße am Ende 5.1 liegt beispielsweise im Bereich zwischen etwa 18 und 30 *µ*m und im Bereich des Schaftes 5.2 im Bereich zwischen etwa 40 und 60 *µ*m. Durch die gröbere Porenstruktur und die härtere Ausbildung der Applikationsspitze 5 am Schaft 5.2 wird u.a. die Zuführung des flüssigen Behandlungsmediums an das Ende 5.1 verbessert, gleichzeitig werden durch die härtere Ausbildung des Schaftes 5.1 die mechanischen Eigenschaften der Applikationsspitze an die Voraussetzungen einer maschinelle Montage wesentlich verbessert angepasst.

Als Material für die Applikationsspitze 5 eignen sich u.a. grundsätzlich poröse, Flüssigkeit leitende Materialien, beispielsweise poröse Kunststoffmaterialien, poröse Keramik, aber auch filz- oder fließartige Materialien. Bevorzugt besteht die Applikationsspitze aus einem gesinterten Material, z.B. aus einem gesinterten polymeren Material, z.B. PE, PP, PVDF, PTFE oder EVA. Wegen der hydrophoben Eigenschaften der polymeren Materialien ist es zweckmäßig, diese Materialien hydrophil zu behandeln.

Weiterhin kann es zweckmäßig sein, die Applikationsspitze 5 an ihrer Umfangsfläche mit wenigstens einer sich über einen Großteil der Länge oder über die gesamte Länge der Applikationsspitze 5 erstreckenden Be- oder Entlüftungsnut zu versehen, um ein insbesondere auch thermisch bedingtes Tropfen oder Auslaufen des Applikators 1 zu vermeiden. Die Applikationsspitze 5 ist weiterhin bevorzugt zumindest an ihrem Ende 5.1, beispielsweise an beiden Enden sich verjüngend ausgebildet, und zwar dann z.B. mit abgerundeter Spitze.

Im zusammengebauten Zustand ist der Einsatz 3 in die Öffnung des Behälters 2 eingesetzt und dort in geeigneter Weise, z.B. durch Verschrauben des mit einem Innengewinde versehenen Abschnittes 4.3 des Mundstückes 4 gesichert, sodass der Einsatz 3 an der Öffnung des Behälters 2 diese Öffnung abdichtend gehalten ist, mit einem röhrchenförmigen Einlass seiner Dosierpumpe 3.1 in den Innenraum des Behälters 2 bzw. in das dort aufgenommene flüssige Behandlungsmedium 8 hineinreicht und mit dem Auslass seiner Dosierpumpe 3.1 im Innenraum des Abschnittes 4.2 des Mundstückes 4 endet. Die Applikationsspitze 5 ist mit ihrem Schaft 5.2 in das Mundstück 4 eingesetzt, und zwar derart, dass sie mit ihrem Ende 5.1 über das in den Figuren 1 und 2 obere Ende des Mundstückes 4 vorsteht und mit ihrem unteren Ende in den Innenraum des Abschnittes 4.2 bzw. in das dort aufgenommene obere Ende des Einsatzes 3 hineinreicht und der Dosierpumpe axial unmittelbar gegenüberliegt. Durch axiales Verschieben der Applikationsspitze 5 gegen die Wirkung einer nicht dargestellten, beispielsweise in der Dosierpumpe 3.1 vorgesehenen Rückstellfeder kann die Dosierpumpe 3.1 betätigt werden, und zwar zur Entnahme einer dosierten Menge des Behandlungsmediums 8 aus dem Behälter 2 und zur Abgabe dieser Menge an die Applikationsspitze 5.

Es versteht sich, dass die Dosierpumpe 3.1 auch andersartig ausgebildet sein kann, und zwar so, dass sie durch Ausübung eines radialen mechanischen Druckes auf das Mundstück 4 und/oder auf den beispielsweise elastisch verformbaren Behälter 2 betätigt wird. Weiterhin ist es auch möglich, anstelle der Dosierpumpe 3.1 ein Dosierventil vorzusehen, welches bei Ausübung des radialen Druckes auf den Applikator 1, insbesondere auf das Mundstück und/oder auf den elastisch ausgebildeten Behälter 2 für die Abgabe einer dosierten Menge des Behandlungsmediums aus dem Behälter öffnet.

Bei Nichtgebrauch der Vorrichtung ist auf das Mundstück 4 die Verschlusskappe 6 aufgesetzt, in der dann auch das über das Mundstück 4 vorstehende Ende 5.1 der Applikationsspitze 5 dicht verschlossen aufgenommen ist.

Zum Applizieren des Behandlungsmediums 8 auf dem zu behandelnden Bereich, z.B. auf die zu behandelnde Haut- oder Gewebepartie wird der zu behandelnden Bereich nach Abnahme der Verschlusskappe 6 und gegebenenfalls nach Betätigung der Dosierpumpe 3.1 oder eines entsprechenden Dosierventils mit dem Ende 5.1 der Applikationsspitze 5 bestrichen. Durch die Dosierpumpe 3.1 bzw. durch das entsprechende Dosierventil wird hierbei nicht nur die dosierte Abgabe des Behandlungsmediums 8 aus dem Innenraum des Behälters 2 erreicht, sondern insbesondere auch verhindert, das Behandlungsmedium von der Applikationsspitze 5 zurück in den Innenraum des Behälters 2 gelangt. Hierdurch wird die Haltbarkeit des Behandlungsmediums 8 im Behälter 2 wesentlich verbessert, vor allem auch eine Verkeimung des im Behälter 2 aufgenommenen Behandlungsmediums 8 vermieden. Mit dem Applikator 1 ist ein unproblematisches, gut dosiertes und auch exakt lokalisiertes Aufbringen des flüssigen Behandlungsmediums 8 auf den zu behandelnden Bereich möglich. Der erfindungsgemäße Applikator ist also insbesondere, aber nicht ausschließlich überall dort bevorzugt verwendbar, wo eine medizinische Behandlung mit einem flüssigen Behandlungsmedium insbesondere auch lokal begrenzt erfolgen soll.

Der Applikator 1 eignet sich zur Aufbringung von wirkstoffhaltigen Lösungen zur Behandlung von Warzen.

Im Detail eignet sich der Applikator 1 mit einem entsprechenden, im Behälter 2 aufgenommenen medizinischen Behandlungsmedium zur Behandlung von Warzen.

Von besonderer Bedeutung für die Funktionstüchtigkeit des Applikators 1 und das Behandlungsergebnis, aber auch für die Stabilität des Behandlungsmediums ist die verwendete Lösung, in der der für die jeweilige medizinische Indikation erforderliche Wirkstoff aufgenommen ist.

Als Lösung eignen sich beispielsweise ölige Lösungen, die dann z.B. mineralische Öle enthalten, wie Paraffinöle, Silikonöle und/oder deren Derivate, pflanzliche Öle, wie z.B. Sonnenblumenöl, aus pflanzlichen Ölen durch Umestern und/oder Teilsynthesen gewonnene Ölprodukte, wie Glycerolester oder andere Ester von Fettsäuren, auch mit Fettalkoholen, Fettalkohole, deren Ether und/oder Ester, enthalten, und zwar jeweils als Einzelbestandteil und/oder in beliebiger Mischung und/oder in unterschiedlichsten Einsatzkonzentrationen.

Derartige Lösungen können weiterhin Zusätze enthalten, wie z.B. Tenside und/oder Wachse, letztere zur Verdickung der Ölphase, Parfümöle und/oder Farbstoffe. Die Tenside sind dann beispielsweise in einen Anteil von etwa 0,1 - 4,0 Gewichts-% und die Wachse in einem Anteil von 0,1 - 1,0 Gewichts-% in der Lösung enthalten, und zwar jeweils bezogen auf die Gesamtmasse der Lösung.

Als Lösung eignen sich weiterhin wässrige, alkoholische Lösungen. Diese enthalten Wasser (aqua dest.) und Alkohole, beispielsweise einwertige und/oder zweiwertige Alkohole wie z.B. Ethanol oder Isopropanol, und zwar z.B. in einer Konzentration von etwa 5 - 70 Gewichts-% bezogen auf das Gesamtgewicht der Lösung, und/oder polymere Ethylen/propylen-Glykole PEG, und/oder mehrwertige, gesättigte und ungesättigte Alkohole, wie z.B. Propylenglykol und/oder Ester und Ether von Alkoholen in einer Konzentration von z.B. 2 - 40 Gewichts-% bezogen auf die Gesamtmasse der jeweiligen Lösung.

Diese alkoholischen Lösungen können ebenfalls weitere Zusätze enthalten, insbesondere auch zur Verbesserung der Stabilität und/oder der physikalischen Eigenschaften und/oder Resorptionsverbesserung der jeweiligen Lösung und damit des Behandlungsmediums. Geeignete Zusätze sind z.B. Tenside zur Verbesserung der Löslichkeit und Oberflächenspreizung, wie z.B. Polysobat 80, bevorzugt in einer Konzentration von 0,5 - 5,0 Gewichts-%, Fettalkohole, deren Ester und Ether, wie z.B. Octyldodecanol, bevorzugt in einer Konzentration von etwa 0,5 - 4,0 Gewichts-%, Verdicker auf Basis von Zellulose, Stärke, Oligosaccharide und/oder Polyacrylate, bevorzugt in eine Konzentration von etwa 0,1 - 0,4 Gewichts-%, Parfümöle und/oder Farbstoffe. Die vorgenannten Angaben in Gewichts-% beziehen sich wiederum auf die Gesamtmasse der jeweiligen Lösung.

Speziell bei den wässrigen alkoholischen Lösungen besteht auch die Möglichkeit, die Wirkstoffe, insbesondere auch die zur Behandlung der jeweiligen Indikation und dabei beispielsweise die zur Behandlung der vorstehend genannten Indikationen erforderlichen Wirkstoffe in liposomaler und/oder nanosomaler Verkapselung vorzusehen.

Geeignet sind weiterhin auch wässrige Lösungen, insbesondere wässrige alkoholische Lösungen der vorstehend genannten Art, die zusätzlich noch polymere Bestandteile zur Filmbildung, d.h. zur Ausbildung eines den jeweiligen Behandlungsbereich abdeckenden polymeren Filmes aufweisen. Diese Lösungen besitzen dann beispielsweise eine Zusammensetzung, wie sie vorstehend für die wässrigen alkoholischen Lösungen angegeben wurde, können aber auch noch andere Lösungsmittel, wie z.B. Ethylacetat vorzugsweise in einer Konzentration von etwa 5,0 - 40 Gewichts-%, Aceton, vorzugsweise in einer Konzentration von etwa 5,0 - 40 Gewichts-% und/oder Filmbrecher, wie z.B. andere Polymere und/oder Co- und Crosspolymere, wie z.B. Polymethacrylate (auch chemisch modifiziert), die geeignet sind, stabile, elastische Filme auf dem behandelten Bereich ausbilden, enthalten.

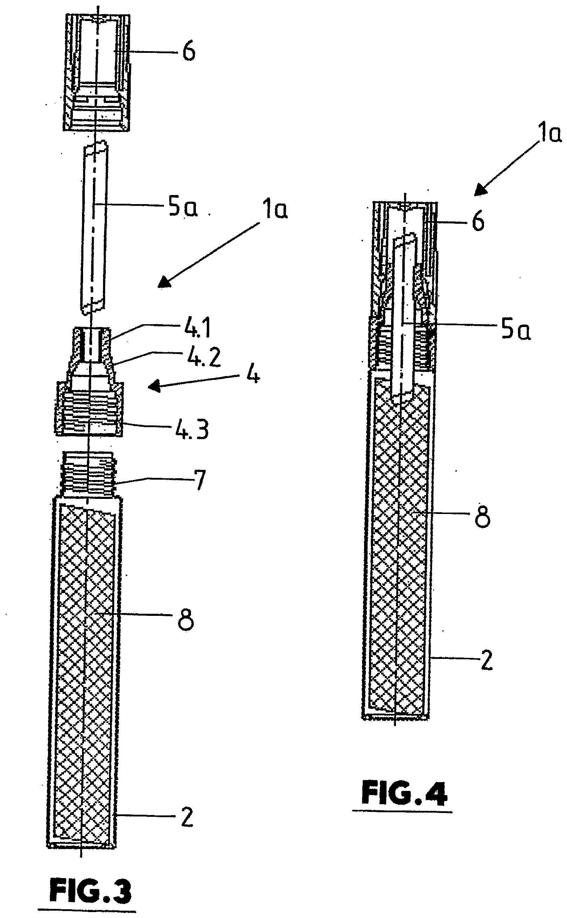

Die Figuren 3 und 4 zeigen eine Vorrichtung 1a, die sich von der Vorrichtung 1 im Wesentlichen nur dadurch unterscheidet, dass der Einsatz 3 mit der Dosierpumpe oder dem Dosierventil entfallen ist und dementsprechend die Applikationsspitze 5a unmittelbar in den Innenraum des Behälters bzw. in das dort aufgenommene Behandlungsmedium 8 hineinreicht. Ansonsten entspricht die Vorrichtung 1a der Vorrichtung 1, sodass in den Figuren 3 und 4 für die der Vorrichtung 1 entsprechenden Elemente dieselben Bezugsziffern wie in den Figuren 1 und 2 verwendet sind. Auch die Vorrichtung 1a) eignet sich insbesondere für die Behandlung der vorstehend genannten Indikationen unter Verwendung eines Behandlungsmediums 8, welches den für die Behandlung der jeweiligen Indikation notwendigen Wirkstoff oder die entsprechende Wirkstoffkombination enthält, und zwar wiederum in einer öligen Lösung, einer wässrigen, alkoholischen Lösung und/oder einer wässrigen, bzw. wässrigen alkoholischen Lösung mit zusätzlichen polymeren Bestandteilen zur Filmbildung.

Die Erfindung wurde voranstehend an Ausführungsbeispielen beschrieben.

So besteht beispielsweise die Möglichkeit, anstelle der Dosierpumpe 3.1 oder eines Dosierventils andere Dosierpumpen und/oder ventilartig wirkende Mittel zur dosierten Abgabe des Behandlungsmediums und/oder zur Verhinderung eines Rückflusses des Behandlungsmediums in den Innenraum des Behälters 2 und/oder zur Behinderung eines Tropfens des Behandlungsmediums vorzusehen. Weiterhin wurde vorstehend davon ausgegangen, dass der Behälter 2 ein röhrchenförmiger oder hülsenartiger Behälter ist. Auch andere Formen sind denkbar, insbesondere kann der zur Aufnahme des Behandlungsmediums dienende Behälter auch tubenartig ausgeführt sein. Um die Applikationsspitze 5 zumindest an der Außenfläche ihres aus dem Mundstück 4 vorstehenden Endes 5.1 möglichst steril zu halten, kann es zweckmäßig sein, in der Verschlusskappe Mittel zur Desinfektion vorzusehen, beispielsweise in Form wenigstens eines in der Kappe angeordneten, mit einem Desinfektionsmittel getränkten Kissens, in welches das Ende 5.1 beim Aufsetzen der Verschlusskappe 6 eintaucht.

### Bezugszeichenliste

- 1, 1a: Vorrichtung bzw. Applikator
- 2: Behälter
- 3: Einsatz
- 3.1: Dosierpumpe
- 4: Mundstück
- 4.1 - 4.3: Abschnitte des Mundstücks 4
- 5, 5a: Applikationsspitze
- 5.1: Applikationsende der Applikationsspitze
- 5.2: Schaft der Applikationsspitze
- 6: Verschlusskappe
- 7: Außengewinde
- 8: Behandlungsmedium oder Behandlungslösung mit medizinischem Wirkstoff

## Patentansprüche

1. Medizinprodukt mit einem Applikator zum gezielten Aufbringen bzw. Applizieren eines flüssigen Behandlungsmediums mit wenigstens einem medizinischen Wirkstoff bei einer äußeren Behandlung eines menschlichen oder tierischen Körpers an einem zu behandelnden Bereich, mit einem stiftartigen Applikator (1, 1a) aus einem Behälter (2), der das Behandlungsmedium enthält und der an einem Ende mit einer Applikationsspitze (5) versehen ist, über die das Behandlungsmedium aus dem Inneren des Behälters (2) durch Überstreichen des zu behandelnden Bereichs auf diesen aufgebracht werden kann, wobei der wenigstens eine medizinische Wirkstoff ein Wirkstoff zur Behandlung von Warzen ist, **dadurch gekennzeichnet dass** die Applikationsspitze (5) aus einem porösen Material besteht und an ihrem zum Aufbringen des Behandlungsmediums dienenden Ende (5.1) eine reduzierte Porosität aufweist.

2. Medizinprodukt nach Anspruch 1, **dadurch gekennzeichnet, dass** das Behandlungsmedium den wenigstens einen Wirkstoff in einer öligen Lösung enthält, wobei die ölige Lösung beispielsweise mindestens ein mineralisches Öl, z.B. Paraffinöl und/oder Silikonöl und/oder wenigstens ein Derivat eines mineralischen Öls und/oder wenigstens ein pflanzliches Öl und/oder Ölprodukt enthält, welches aus wenigstens einem pflanzlichen Öl durch Umestern und/oder Teilsynthese gewonnen ist.

3. Medizinprodukt nach Anspruch 2, **dadurch gekennzeichnet, dass** als Ölprodukt Glycerolester oder andere Ester von Fettsäuren mit Fettalkoholen, Fettalkohole und/oder deren Ether und Ester verwendet ist, auch in beliebiger Mischung und in beliebigen Anteilen.

4. Medizinprodukt nach Anspruch 3, **dadurch gekennzeichnet, dass** die ölige Lösung zumindest einen Zusatz in Form von Tensiden, vorzugsweise mit einem Anteil von etwa 0,1 - 4,0 Gewichts-%, bezogen auf die Gesamtmasse der Lösung, und/oder in Form von Wachsen, vorzugsweise mit einem Anteil von etwa 0,1 - 1,0 Gewichts-% bezogen auf die Gesamtmasse der Lösung, und/oder Parfümöle und/oder Farbstoffe enthält.

5. Medizinprodukt nach einem der Ansprüche 1 - 4, **dadurch gekennzeichnet, dass** das Behandlungsmedium den wenigstens einen medizinischen Wirkstoff in einer wässrigen, alkoholischen Lösung enthält, wobei die wässrige alkoholische Lösung beispielsweise ein- oder mehrwertige Alkohole, wie z.B. Ethanol und/oder Isopropanol und/oder Propylenglykol und/oder deren Ester und/oder Ether und/oder Ethanol und/oder Isopropanol in einer Konzentration von etwa 5,0 - 70 Gewichts-%, bezogen auf die Gesamtmasse der Lösung enthält.

6. Medizinprodukt nach Anspruch 5, **dadurch gekennzeichnet, dass** die wässrige alkoholische Lösung die mehrwertigen, gesättigten und/oder ungesättigten Alkohole, wie z.B. Propylenglykol und/oder deren Ester und/oder Ether in einer Konzentration von etwa 2,0 - 40 Gewichts-%, bezogen auf die Gesamtmasse der Lösung enthält, und/oder
dass die wässrige alkoholische Lösung den wenigstens einen medizinischen Wirkstoff in lioposomaler und/oder nanosomaler Verkapselung enthält,
und/oder
dass die wässrige alkoholische Lösung einen Film bildenden Bestandteil, vorzugsweise einen Film bildenden polymeren Bestandteil enthält, wobei als Film bildender Bestandteil beispielsweise Ethylacetat, vorzugsweise in einer Konzentration von etwa 5,0 - 40 Gewichts-%, und/oder beispielsweise Aceton, vorzugsweise in einer Konzentration von etwa 5,0 - 40 Gewichts%, jeweils bezogen auf die Gesamtmasse der Lösung, und/oder Polymere und/oder Crosspolymere, wie z.B. Polymethacrylate, vorzugsweise auch chemisch modifiziert verwendet sind.

7. Medizinprodukt nach einem der Ansprüche 5 oder 6, **dadurch gekennzeichnet, dass** die wässrige alkoholische Lösung als Zusatz Tenside, vorzugsweise in einer Konzentration von etwa 0,5 - 5 Gewichts-%, bezogen auf die Gesamtmasse der Lösung, und/oder Fettalkohole und/oder deren Ester und/oder Ether, vorzugsweise in einer Konzentration von etwa 0,5 - 4,0 Gewichts%, bezogen auf die Gesamtmasse der Lösung enthält.

8. Medizinprodukt nach Anspruch 7, **dadurch gekennzeichnet**, dassPolysorbat80 und/oder Octyldodecanol als Zusatz verwendet sind.

9. Medizinprodukt nach einem der Ansprüche 5 - 8, **dadurch gekennzeichnet, dass** die wässrige alkoholische Lösung als Zusatz Verdicker auf Basis von Cellulose und/oder Stärke, beispielsweise Oligosaccharide und/oder Polyacrylate, vorzugsweise in einer Konzentration von etwa 0,1 - 0,4 Gewichts-%, bezogen auf die Gesamtmasse der Lösung enthält.

10. Medizinprodukt nach einem der Ansprüche 1 - 9, **gekennzeichnet durch** eine Applikationsspitze aus einem porösen gesinterten Material, z.B. aus einem gesinterten polymeren Material.

11. Medizinprodukt nach einem der Ansprüche 1 - 10, **gekennzeichnet durch** eine Applikationsspitze (5), die an ihrem zum Aufbringen des Behandlungsmediums auf die Haut dienenden Ende mit reduzierter Härte ausgebildet ist.

12. Medizinprodukt nach einem der Ansprüche 1 - 11, **dadurch gekennzeichnet, dass** die Applikationsspitze (5) aus PE, PP, PVDF, PTFE und/oder EVA besteht.

## Claims

1. A medical device with an applicator for the targeted delivery or application of a liquid treatment medium with at least one medically active substance for an external treatment of a human or animal body to an area to be treated, with a pen-type applicator (1, 1a) from a reservoir (2) which contains the treatment medium and which is provided with an application tip (5) at one end thereof, via which the treatment medium can be applied to the area for treatment from the interior of the container (2) by sweeping over said area with the applicator, wherein the at least one medically active substance is a substance for treating warts, **characterized in that** the application tip (5) consists of a porous material, and the end (5.1) thereof that is used for applying the treatment medium has reduced porosity.

2. The medical device according to claim 1, **characterized in that** the treatment medium contains the at least one active substance in an oily solution, wherein the oily solution contains for example at least one mineral oil, e.g., paraffin oil and/or silicone oil and/or at least one derivative of a mineral oil and/or at least one vegetable oil and/or oil product which is obtained from at least one vegetable oil by transesterification and/or partial synthesis.

3. The medical device according to claim 2, **characterized in that** glycerol ester or other esters of fatty acids with fatty alcohols, fatty alcohols and/or ethers and esters thereof are used as the oil product, also in any mixture and in any proportions.

4. The medical device according to claim 3, **characterized in that** the oily solution contains at least one additive in the form of surfactants, preferably in a proportion of about 0.1 - 4.0 % by weight relative to the total mass of the solution, and/or in form of waxes, preferably in a proportion of about 0.1 - 1.0 % by weight relative to the total mass of the solution, and/or perfume oils and/or dyes.

5. The medical device according to any one of claims 1 - 4, **characterized in that** the treatment medium contains the at least one medically active substance in an aqueous alcohol solution, wherein the aqueous alcohol solution contains for example monovalent or polyvalent alcohols such as e.g. ethanol and/or isopropanol and/or propylene glycol and/or esters and/or ethers thereof, and/or ethanol and/or isopropanol in a concentration of about 5.0 - 70 % by weight relative to the total mass of the solution.

6. The medical device according to claim 5, **characterized in that** the aqueous alcohol solution contains the polyvalent, saturated and/or unsaturated alcohols such as propylene glycol and/or esters and/or ethers thereof in a concentration of about 2.0 - 40 % by weight relative to the total mass of the solution
and/or
**in that** the aqueous alcohol solution contains the at least one medically active substance in liposomal and/or nanosomal encapsulation,
and/or
**in that** the aqueous alcohol solution contains a film-forming constituent, preferably a polymeric film-forming constituent, wherein for example ethyl acetate, preferably in a concentration of about 5.0 - 40 % by weight, and/or for example acetone, preferably in a concentration of about 5.0 - 40 % by weight relative in each case to the total mass of the solution, and/or polymers and/or cross polymers such as for example polymethacrylates, preferably also chemically modified, are used as the film-forming constituent.

7. The medical device according to one of claims 5 or 6, **characterized in that** the aqueous alcohol solution contains as additive surfactants, preferably in a concentration of about 0.5 - 5 % by weight relative to the total mass of the solution, and/or fatty alcohols and/or esters and/or ethers thereof, preferably in a concentration of about 0.5 - 4.0 % by weight relative to the total mass of the solution.

8. The medical device according to claim 7, **characterized in that** polysorbate 80 and/or octyl dodecanol are used as additive.

9. The medical device according to any one of claims 5 - 8, **characterized in that** the aqueous alcohol solution contains as additive thickeners on the basis of cellulose and/or starch, for example oligosaccharides and/or polyacrylates, preferably in a concentration of about 0.1 - 0.4 % by weight relative to the total mass of the solution.

10. The medical device according to any one of claims 1 - 9, **characterized by** an application tip made of a porous sintered material, for example a sintered polymeric material.

11. The medical device according to any one of claims 1 - 10, **characterized by** an application tip (5) of which the end that is used to apply the treatment medium to the skin is constructed with reduced hardness.

12. The medical device according to any one of claims 1 - 11, **characterized in that** the application tip (5) consists of PE, PP, PVDF, PTFE and/or EVA.

## Revendications

1. Produit médical comprenant un applicateur pour l'application ciblée d'un agent de traitement liquide comprenant au moins une substance active médicale lors d'un traitement extérieur d'un corps humain ou animal sur une zone à traiter, avec un applicateur en forme de bâtonnet (1, 1a) à partir d'un récipient (2) qui contient l'agent de traitement et qui est doté d'une pointe d'application (5) à une extrémité, par le biais de laquelle l'agent de traitement peut être appliqué hors de l'intérieur du récipient (2) par passage en couches sur la zone à traiter, dans lequel l'au moins une substance active médicale est une substance active pour traiter des verrues, **caractérisé en ce que** la pointe d'application (5) se compose d'un matériau poreux et présente une porosité réduite sur son extrémité (5.1) servant à appliquer l'agent de traitement.

2. Produit médical selon la revendication 1, **caractérisé en ce que** l'agent de traitement contient l'au moins une substance active dans une solution huileuse, dans lequel la solution huileuse contient par exemple au moins une huile minérale, par exemple de l'huile de paraffine et/ou de l'huile de silicone et/ou au moins un dérivé d'une huile minérale et/ou au moins une huile végétale et/ou un produit oléique, qui est extrait d'au moins une huile végétale par échange d'esters et/ou synthèse partielle.

3. Produit médical selon la revendication 2, **caractérisé en ce que** de l'ester de glycérol ou d'autres esters d'acides gras avec alcools gras, des alcools gras et/ou leurs éthers et esters sont employés en tant que produit oléique, également en mélange au choix et en teneurs au choix.

4. Produit médical selon la revendication 3, **caractérisé en ce que** la solution huileuse contient au moins un additif sous forme d'agents tensio-actifs, de préférence avec une teneur d'environ 0,1 - 4,0 % en poids rapporté à la masse totale de la solution, et/ou sous forme de cires, de préférence avec une teneur d'environ 0,1 - 1,0 % en poids rapporté à la masse totale de la solution et/ou d'huiles de parfum et/ou de colorants.

5. Produit médical selon l'une des revendications 1 - 4, **caractérisé en ce que** l'agent de traitement contient l'au moins une substance active médicale dans une solution alcoolique aqueuse, dans lequel la solution alcoolique aqueuse contient par exemple des alcools à monovalents ou plurivalents, comme par ex. de l'éthanol et/ou de l'isopropanol et/ou du propylène glycol et/ou leurs esters et/ou éthers et/ou de l'éthanol et/ou de l'isopropanol dans une concentration d'environ 5,0 - 70 % en poids rapporté à la masse totale de la solution.

6. Produit médical selon la revendication 5, **caractérisé en ce que** la solution alcoolique aqueuse contient les alcools polyvalents, saturés et/ou insaturés, comme par exemple du propylène glycol et/ou leurs esters et/ou éthers dans une concentration d'environ 2,0 - 40 % en poids rapporté à la masse totale de la solution,
et/ou
**que** la solution alcoolique aqueuse contient l'au moins une substance active médicale en encapsulage lioposomal et/ou nanosomal,
et/ou
**que** la solution alcoolique aqueuse contient une composante formant un film, de préférence une composante polymère formant un film, dans lequel par exemple de l'éthyl-acétate, de préférence dans une concentration d'environ 5,0 - 40 % en poids et/ou par exemple de l'acétone, de préférence dans une concentration d'environ 5,0 - 40 % en poids, respectivement rapportés à la masse totale de la solution, et/ou des polymères et/ou des polymères croisés, comme par exemple du polyméthacrylate, de préférence également modifiés chimiquement sont employés en tant que composante polymère formant un film.

7. Produit médical selon l'une des revendications 5 ou 6, **caractérisé en ce que** la solution alcoolique aqueuse contient en tant qu'additifs des agents tensio-actifs, de préférence dans une concentration d'environ 0,5 - 5 % en poids, rapportés à la masse totale de la solution, et/ou des alcools gras et/ou leurs esters et/ou éthers, de préférence dans une concentration d'environ 0,5 - 4,0 % en poids rapporté à la masse totale de la solution.

8. Produit médical selon la revendication 7, **caractérisé en ce que** du polysorbate 80 et/ou de l'octyldodécanol sont employés en tant qu'additif.

9. Produit médical selon l'une des revendications 5 - 8, **caractérisé en ce que** la solution alcoolique aqueuse contient en tant qu'additifs des épaississants à base de cellulose et/ou d'amidon, par exemple des oligosaccharides et/ou des polyacrylates, de préférence dans une concentration d'environ 0,1 - 0,4 % en poids rapporté à la masse totale de la solution.

10. Produit médical selon l'une des revendications 1 - 9, **caractérisé par** une pointe d'application en un matériau fritté poreux, par exemple dans un matériau polymère fritté.

11. Produit médical selon l'une des revendications 1 - 10, **caractérisé par** une pointe d'application (5) qui est formée avec une dureté réduite sur son extrémité servant à l'application de l'agent de traitement sur la peau.

12. Produit médical selon l'une des revendications 1 - 11, **caractérisé par** une pointe d'application (5) qui se compose de PE, PP, PVDF, PTFE et/ou EVA.
